# DEMANDE DE BREVET EUROPEEN

(11) **EP 4 289 386 A1**
(43) Date de publication de la demande: **13.12.2023**
(21) Numéro de dépôt: 23177511.5
(22) Date de dépôt: 06.06.2023
(51) Int. Cl.: A61B 90/00, A61B 34/30, A61C 9/00, G06N 20/00, A61B 34/37, A61B 34/10

(54) **PROCÉDÉ DE COMMANDE D'UN BRAS ROBOTISÉ POUR IMAGERIE INTRA-ORALE**

(30) Priorité: 07.06.2022 FR 2205445
(71) Demandeur: Querbes, Olivier, 31570 Vallesvilles (FR); Querbes-Duret, Véronique, 31570 Vallesvilles (FR)
(72) Inventeur: Querbes, Olivier, 31570 Vallesvilles (FR); Querbes-Duret, Véronique, 31570 Vallesvilles (FR)
(74) Mandataire: Bringer IP

(57) **Abrégé**

L'invention concerne un procédé de commande de commande d'un bras (102) robotisé comprenant une caméra (104) intraorale, comprenant une étape de commande du déplacement du bras robotisé en fonction d'une portion de trajectoire déterminée, et une étape de capture d'images de la cavité orale par la caméra, caractérisé en ce que le procédé comprend en outre une étape préliminaire de définition d'un ensemble de tissus intra-oraux à reconstruire, une étape préliminaire de détermination d'au moins une portion de la trajectoire du bras robotisé en fonction d'une position de départ de la caméra et de l'ensemble de tissus à reconstruire, une étape de segmentation sémantique de chaque pixel de chaque image capturée pour déterminer un ensemble de tissus reconstruits, et après chaque ajout d'un tissu dans l'ensemble de tissus reconstruits, une étape de détermination d'une nouvelle portion de trajectoire selon l'ensemble de tissus reconstruits et l'ensemble de tissus à reconstruire.

## Description

### Domaine technique de l'invention

L'invention concerne un procédé de commande d'un bras robotisé pour une imagerie intra-orale. En particulier, l'invention concerne un procédé permettant le calcul d'une trajectoire du bras robotisé portant une caméra de façon à capter des images intraorales, afin par exemple de réaliser une reconstruction 3D d'éléments de la cavité orale dans laquelle se déplace le bras robotisé, en particulier la reconstruction de tout ou partie des tissus dentaires de la cavité orale.

### Arrière-plan technologique

La reconstruction 3D d'une cavité orale permet de générer un modèle 3D permettant notamment d'identifier chacune des dents et des tissus de la cavité orale. Cette cavité orale reconstruite est utilisée comme base pour divers traitements dentaires (prothétiques, esthétiques, etc).

Dans le cadre de ces traitements dentaires, la prise d'empreinte de l'arcade dentaire se faisait auparavant par une prise d'empreinte physique à base d'alginate, mais cette technique est peu à peu remplacée par des reconstructions tridimensionnelles numériques obtenues grâce à des caméras intra-orales. Ces reconstructions tridimensionnelles numériques des arcades dentaires permettent d'utiliser les outils de Conception Assistée par Ordinateur (CAO), ce qui offrent les avantages d'élargir et de simplifier les possibilités de traitement et/ou de conception de prothèse.

Une caméra intraorale est un dispositif composé d'un ou plusieurs capteurs optiques, qui acquièrent des images intraorales et les transforment en une reconstruction tridimensionnelle des tissus intraoraux ciblés (arcade dentaire partielle ou complète, palais, gencive, dent isolée, etc). Les images sont transmises en temps réel à une unité de traitement, qui calcule la surface tridimensionnelle correspondante, et renvoie en temps réel sur un écran la reconstruction 3D actuelle des tissus afin de montrer à l'utilisateur ce qu'il a correctement acquis jusque-là.

De plus en plus de caméras intraorales sortent régulièrement sur le marché, mais elles partagent toutes un même inconvénient : un opérateur humain doit intervenir pour manipuler la caméra dans la bouche du patient, afin d'ajuster continuellement son geste à la reconstruction tridimensionnelle temps réel présentée, pour la corriger ou la compléter jusqu'à acquisition complète de la zone anatomique désirée.

Des solutions ont été proposées pour automatiser le déplacement de la caméra dans la bouche du patient, en particulier par génération d'une trajectoire de déplacement en fonction de paramètres anatomiques de la bouche du patient.

Ces solutions présentent différents inconvénients : en premier lieu, elles sont contraignantes pour l'opérateur qui doit positionner le bras robotisé à sa position initiale de façon correcte pour s'assurer que la trajectoire corresponde bien au patient.

En outre, la trajectoire calculée est liée à des paramètres anatomiques limités et ne permet pas de s'adapter à tout type d'anatomie particulière, par exemple une dent perdue ou une anomalie dentaire de type manque d'une ou plusieurs dents, dent surnuméraire, dents ou cavité orale de dimensions trop différentes de la normale, etc.

Ces solutions sont en outre utilisées pour un parcours complet de la cavité orale ou au moins de l'arcade dentaire et ne permettent pas de sélection de portions d'arcade dentaire, ou de retour en arrière pour obtenir de meilleures images.

Enfin, ces trajectoires prédéfinies nécessitent une immobilisation complète de la tête du patient car elles ne prévoient pas d'éventuels mouvements, même minimes de la cavité orale du patient, même si des capteurs haptiques sont généralement présents pour éviter toute blessure.

Les inventeurs ont donc cherché une solution permettant de pallier ces inconvénients en fournissant un procédé de commande automatique.

### Objectifs de l'invention

L'invention vise à fournir un procédé de commande d'un bras robotisé comprenant une caméra configurée pour la capture d'images intraorales palliant les inconvénients cités.

L'invention vise en particulier à fournir, dans au moins un mode de réalisation, un procédé de commande permettant la reconstruction 3D de portions prédéfinies de la cavité orale.

L'invention vise aussi à fournir, dans au moins un mode de réalisation de l'invention, un procédé de commande permettant de s'adapter aux différentes anatomies existantes.

L'invention vise aussi à fournir, dans au moins un mode de réalisation de l'invention, un procédé de commande facilitant la mise en place par un praticien.

L'invention vise aussi à fournir, dans au moins un mode de réalisation de l'invention, un procédé de commande dont la trajectoire du bras robotisé peut être recalculée en temps réel.

L'invention vise aussi à fournir, dans au moins un mode de réalisation de l'invention, un système de commande d'un bras robotisé comprenant une caméra configurée pour la capture d'images intraorales.

### Exposé de l'invention

Pour ce faire, l'invention concerne un procédé de commande d'un bras robotisé comprenant une caméra configurée pour la capture d'images intraorales, le procédé comprenant une étape de détermination d'au moins une portion d'une trajectoire dudit bras robotisé à l'intérieur d'une cavité orale, une étape de commande du déplacement du bras robotisé dans la cavité orale en fonction de ladite portion de trajectoire déterminée, et une étape de capture d'images de la cavité orale par la caméra du bras robotisé,
caractérisé en ce que le procédé comprend en outre les étapes suivantes :
- une étape préliminaire de définition d'un ensemble de tissus intra-oraux dont au moins une image doit être captée par la caméra, dit ensemble de tissus à reconstruire,
- une étape préliminaire de détermination d'au moins une portion de la trajectoire du bras robotisé en fonction d'une position de départ de la caméra et de l'ensemble de tissus à reconstruire,
- une étape de segmentation sémantique de chaque pixel de chaque image capturée de sorte à déterminer un ensemble de tissus reconstruits parmi l'ensemble de tissus à reconstruire,
- après chaque ajout d'un tissu dans l'ensemble de tissus reconstruits, une étape de détermination d'une nouvelle portion de trajectoire en fonction de l'ensemble de tissus reconstruits et l'ensemble de tissus à reconstruire.

Un procédé selon l'invention permet donc un recalcul répété de la trajectoire lors de la commande du robot afin de s'adapter en temps réel à la cavité orale, aux particularités anatomiques et aux mouvements éventuels du patient, en permettant un changement de stratégie au fur et à mesure de l'avancée de la reconstruction.

Le procédé permet d'autre part de s'adapter à l'ensemble des tissus à reconstruire formant l'objectif visé par la commande du bras robotisé. La trajectoire est ainsi fixée en lien avec ces objectifs qui peuvent varier d'un patient à un autre, plutôt que de viser une reconstruction systématiquement complète de la cavité orale et/ou de l'arcade dentaire comme c'est le cas dans l'art antérieur. L'objectif correspond à l'ensemble de tissus à reconstruire définis dans l'étape préliminaire de définition : cette étape préliminaire de définition peut être réalisée par exemple par un opérateur humain (définition manuelle), éventuellement assisté par un dispositif informatique (définition semi-automatique), ou bien entièrement réalisée par un dispositif informatique (définition automatique) grâce à des données qui lui sont fournies. Un objectif complémentaire est de minimiser la durée de la reconstruction, en particulier la durée pendant laquelle la caméra est agencée dans la cavité orale du patient, en mettant en oeuvre une trajectoire la plus optimale possible.

On entend par portion de trajectoire une consigne de déplacement permettant de se déplacer d'un tissu à reconstruire à un autre tissu à reconstruire de l'ensemble de tissu à reconstruire pour la capture d'images, et par la trajectoire la somme des portions permettant de capturer l'image de tous les tissus de l'ensemble des tissus à reconstruire.

La prise en compte au fur et à mesure des tissus déjà reconstruits permet d'offrir une liberté de reconstruction, par exemple en permettant un démarrage de la trajectoire à n'importe quelle position et en permettant de reconstruire uniquement des morceaux disjoints de la cavité orale, par exemple en choisissant un ensemble de dents que l'on veut reconstruire.

La segmentation sémantique est de façon connue obtenue par exemple par un algorithme d'apprentissage machine, notamment d'apprentissage profond, qui permet d'associer à chaque pixel d'une image une étiquette, une catégorie ou un label, cette étiquette/catégorie/label correspondant à un objet ou un élément généralement réel présent sur l'image. Un exemple de segmentation sémantique mis en oeuvre par un réseau neuronal totalement convolutif est décrit par exemple dans la publication « LONG, Jonathan, SHELHAMER, Evan, et DARRELL, Trevor. Fully convolutional networks for semantic segmentation. In : Proceedings of the IEEE conférence on computer vision and pattern recognition. 2015. p. 3431-3440. »

L'invention peut également être utilisée pour la reconstruction 3D d'un modèle 3D réel, par exemple un moulage d'une cavité orale, notamment d'une arcade dentaire, dans le cadre de la numérisation de moulage existants. La caméra configurée pour la capture d'images intraorales est appelée couramment caméra intraorale et peut être de différents types.

Avantageusement et selon l'invention, la trajectoire ou la portion de trajectoire est déterminée en fonction d'une trajectoire modèle prédéfinie correspondant à au moins une trajectoire classique utilisée par un opérateur humain.

Selon cet aspect de l'invention, la trajectoire est établie en partie en prenant compte de trajectoires classique telles qu'elles sont mises en oeuvre par un praticien tel qu'un dentiste. La trajectoire peut ainsi être adaptée notamment pour permettre une meilleure qualité de prise de vue, en prenant en compte les trajectoires classiques pour lesquelles le praticien sait comment obtenir des images de qualité par son expérience professionnelle.

En particulier, selon au moins une variante de l'invention, les trajectoires peuvent être définies selon des règles heuristiques par rapport à la situation courante du robot. Par exemple, lorsque le robot survole la face occlusale des molaires gauches, une portion de trajectoire peut être définie comme une rotation de X radians selon tel axe et une translation de Y mm selon tel axe, jusqu'à atteindre la face vestibulaire des molaires gauches. La segmentation sémantique permet de vérifier que la face vestibulaire est bien atteinte suite au déplacement. En cours de déplacement, la segmentation sémantique peut aussi permettre de déterminer une anomalie de trajectoire et déclencher un recalcul de trajectoire.

Avantageusement, le procédé selon l'invention comprend une étape préalable d'entraînement d'un réseau neuronal d'une unité de commande du robot par apprentissage par renforcement, ladite étape préalable d'entraînement prenant comme données d'entrée une pluralité de trajectoires classiques utilisées par un opérateur humain.

Selon cet aspect de l'invention, l'apprentissage par renforcement permet au bras robotisé de suivre des portions de trajectoire ou des trajectoires non définies par un opérateur humain ou non programmées à l'avance mais évoluer selon les données de sortie d'un réseau neuronal entraîné sur une pluralité de trajectoires classiques, pouvant ainsi s'adapter à tout type de cavités intra-orales, en particulier sur tout type d'arcades dentaires. Une méthode d'apprentissage par renforcement pouvant être utilisée dans ce contexte est par exemple décrite dans la publication « CHAPLOT, Devendra Singh, GANDHI, Dhiraj Prakashchand, GUPTA, Abhinav, et al. Object goal navigation using goal-oriented semantic exploration. Advances in Neural Information Processing Systems, 2020, vol. 33, p. 4247-4258. »

Avantageusement et selon l'invention, la trajectoire ou la portion de trajectoire est déterminée en fonction d'un modèle 3D prédéfini de la cavité orale.

Selon cet aspect de l'invention, l'utilisation d'un modèle 3D prédéfini peut permettre de faciliter la détermination de la trajectoire. En particulier, le modèle 3D prédéfini peut être un modèle simplifié, par exemple obtenu à l'aide de photos comme le propose le demandeur dans la demande de brevet WO2021245274A1.

Avantageusement et selon l'invention, au moins une portion de trajectoire comprend un déplacement jusqu'à une détection d'un tissu prédéterminé par la caméra intraorale, ledit tissu prédéterminé étant détecté par segmentation sémantique dans au moins une image de ladite caméra intraorale.

Selon cet aspect de l'invention, le procédé de commande permet de mettre en oeuvre des trajectoires conditionnelles, dans lequel la détection d'un tissu particulier déclenche un nouveau calcul de trajectoire ou l'arrêt du procédé de commande. Par exemple, le procédé de commande peut permettre une reconstruction d'un palais de la cavité orale du patient en prévoyant une trajectoire allant d'une dent à la dent opposée sur la même arcade dentaire, et de recalculer une nouvelle trajectoire une fois la dent opposée détectée par la segmentation sémantique, indiquant que la totalité du palais de dent à dent a été reconstruite.

Avantageusement, un procédé selon l'invention comprend une étape de détermination d'une nouvelle trajectoire suite à une détection d'un tissu non prévu par la caméra intraorale, ledit tissu non prévu étant détecté par segmentation sémantique dans au moins une image de ladite caméra intraorale.

Selon cet aspect de l'invention, un recalcul de la trajectoire peut être réalisé lorsqu'un tissu non prévu, tel qu'un trou ou une dent surnuméraire, est détecté de sorte que la portion de trajectoire prévue à l'origine n'est plus adaptée pour reconstruire les tissus de l'ensemble de tissus à reconstruire. L'étape peut également comprendre une suppression d'un ou plusieurs tissus dans l'ensemble de tissus à reconstruire, par exemple par détection d'un trou en l'absence d'une dent.

Avantageusement et selon l'invention, l'ensemble de tissus à reconstruire comprend au moins un tissu inclus dans la liste suivante :
- une dent,
- une gencive,
- un palais,
- une prothèse dentaire,
- une préparation dentaire,
- un pilier implantaire.

Selon cet aspect de l'invention, la diversité de tissus à reconstruire permet la mise en oeuvre du procédé de commande pour la reconstruction de différentes zones de la cavité orale, pour différents résultats, en particulier pour la génération d'un modèle 3D de la cavité orale et/ou de l'arcade dentaire. La préparation dentaire désigne la dent préparée pour recevoir une couronne dentaire. Le pilier implantaire est aussi plus connu sous le nom de *scanbody* en anglais. L'invention ne se limite pas à ces tissus.

L'invention concerne également un système d'imagerie comprenant un bras robotisé comprenant une caméra configurée pour la capture d'images intraorales, caractérisé en ce qu'il comprend une unité de commande, configurée pour recevoir les images capturées par la caméra intraorale, et commander le bras robotisé selon un procédé de commande selon l'invention.

L'invention concerne également un procédé de commande et un système d'imagerie caractérisés en combinaison par tout ou partie des caractéristiques mentionnées ci-dessus ou ci-après.

### Liste des figures

D'autres buts, caractéristiques et avantages de l'invention apparaîtront à la lecture de la description suivante donnée à titre uniquement non limitatif et qui se réfère aux figures annexées dans lesquelles :
[Fig. 1] est un schéma dentaire représentant la numérotation de dents humaines selon le système de notation de la Fédération Dentaire Internationale,
[Fig. 2] est une vue schématique d'un système d'imagerie selon un mode de réalisation de l'invention,
[Fig. 3] est une vue schématique d'un procédé de commande selon un mode de réalisation de l'invention,
[Fig. 4] est une vue schématique d'une arcade dentaire et d'une trajectoire suivie par la caméra intraorale lors de la mise en oeuvre d'un procédé de commande selon un premier mode de réalisation de l'invention,
[Fig. 5] est une vue schématique d'une arcade dentaire et d'une trajectoire suivie par la caméra intraorale lors de la mise en oeuvre d'un procédé de commande selon un deuxième mode de réalisation de l'invention.

### Description détaillée d'un mode de réalisation de l'invention

Sur les figures, les échelles et les proportions ne sont pas strictement respectées et ce, à des fins d'illustration et de clarté.

En outre, les éléments identiques, similaires ou analogues sont désignés par les mêmes références dans toutes les figures.

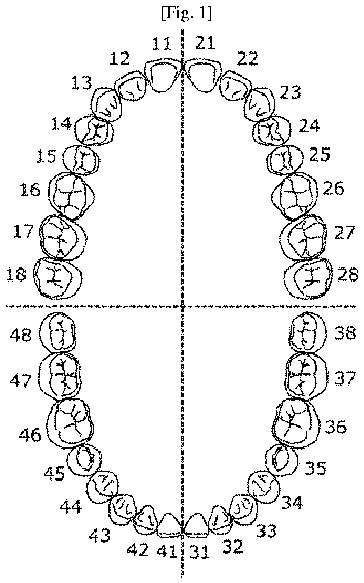

La figure 1 est un schéma représentant une dentition humaine adulte, dans lequel chaque dent est associé à sa notation selon la notation de la Fédération Dentaire Internationale (*FDI World Dental Federation notation* en anglais). Dans cette notation, chaque dent est repérée par deux chiffres de notation ; La dentition est séparée en quatre quadrants, et le quadrant dans laquelle se trouve la dent correspond au premier chiffre de la notation : quadrant 1 en haut à gauche, quadrant 2 en haut à droite, quadrant 3 en bas à droite, quadrant 4 en bas à gauche (« droite » et « gauche » étant entendu du point de vue d'un dentiste observant la dentition d'un patient). Le deuxième chiffre de la notation indique la dent correspondante, dans le quadrant, de 1 l'incisive centrale à 8 la dent de sagesse.

Il existe aussi une notation de la Fédération Dentaire Internationale pour les dents temporaires, non détaillée ici.

Le procédé de commande selon l'invention permet de commander un bras robotisé afin d'obtenir des images intraorales de tissus à reconstruire, par exemple de dents à reconstruire.

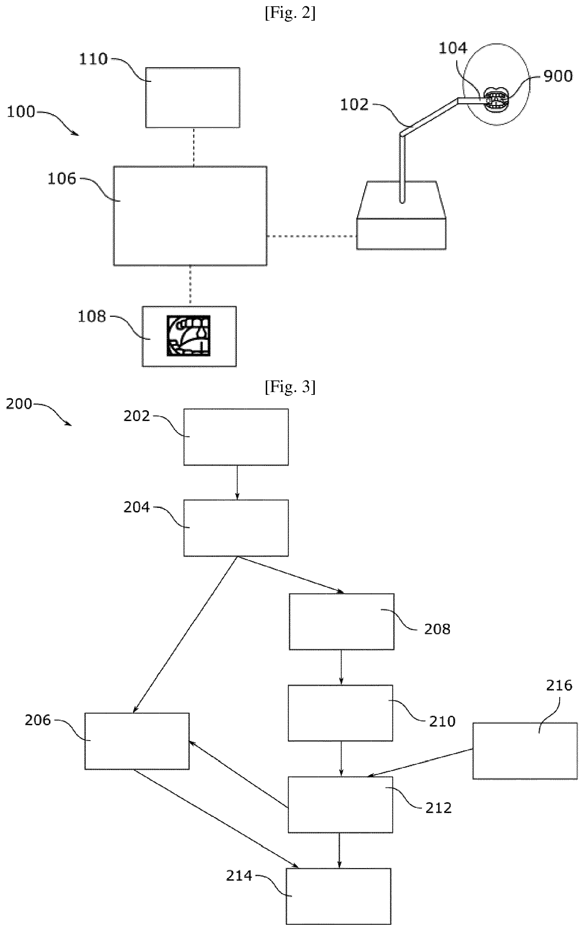

La figure 2 représente schématiquement un système 100 d'imagerie selon un mode de réalisation de l'invention, en particulier configuré pour mettre en oeuvre un procédé de commande selon un mode de réalisation de l'invention.

Le système 100 d'imagerie comprend un bras 102 robotisé, par exemple un bras robotisé de type six axes, comprenant à une de ces extrémités une caméra 104 configuré pour la capture d'images intraorales dans la cavité 900 intra-orale d'un patient, autrement dit une caméra intraorale. La caméra 104 intraorale peut être une caméra telle qu'utilisée actuellement sur le marché pour un scan intraoral manuel, et permet l'acquisition en temps réel de données brutes sur les tissus dentaires, ces données pouvant être (suivant le type de caméra) des images 2D, des reconstructions 3D partielles, ou autres.

Le système 100 d'imagerie comprend également une unité 106 de commande, permettant d'une part le pilotage et l'asservissement du bras 102 robotisé, la réception des données brutes provenant de la caméra 104 intraorale, et le traitement de ces données brutes.

Le traitement des données brutes peut par exemple être une combinaison en temps réel pour produire un modèle 3D temps réel incrémental, qui peut être monochrome ou en couleurs. Une étape optionnelle permet l'application de traitements numériques sur le dernier modèle 3D incrémental pour produire une reconstruction 3D finale de haute précision.

Selon certains modes de réalisation, les données brutes ou traitées peuvent être transmises à un système 108 d'affichage tel qu'un écran d'affichage, permettant un contrôle visuel du déroulement de la capture d'image et de la commande du bras 102 robotisé.

Les données numériques brutes ou issues du traitement par l'unité de commande sont stockées dans une unité 110 de stockage mémoire.

La figure 3 représente schématiquement un procédé 200 de commande selon un mode de réalisation de l'invention.

Le procédé comprend notamment les étapes suivantes :
- une étape 202 préliminaire de définition d'un ensemble de tissus intra-oraux dont au moins une image doit être captée par la caméra, dit ensemble de tissus à reconstruire,
- une étape 204 préliminaire de détermination d'au moins une portion de la trajectoire du bras robotisé en fonction d'une position de départ de la caméra et de l'ensemble de tissus à reconstruire,
- une étape 206 de commande du déplacement du bras robotisé dans la cavité orale en fonction de ladite portion de trajectoire déterminée,
- une étape 208 de capture d'images de la cavité orale par la caméra du bras robotisé
- une étape 210 de segmentation sémantique de chaque pixel de chaque image capturée de sorte à déterminer un ensemble de tissus reconstruits parmi l'ensemble de tissus à reconstruire,
- après chaque ajout d'un tissu dans l'ensemble de tissus reconstruits, une étape 212 de détermination d'une nouvelle portion de trajectoire en fonction de l'ensemble de tissus reconstruits et l'ensemble de tissus à reconstruire.

Lorsque l'ensemble des tissus sont reconstruits, une étape 214 finale du procédé est mise en oeuvre, qui permet par exemple le retrait de la caméra orale de la cavité orale du patient.

L'étape 206 de commande du bras robotisé s'effectue en parallèle de la capture et la segmentation d'image, en fonction de ladite au moins une portion de trajectoire déterminée par l'étape 204 préliminaire en fonction de la position de départ, ou en fonction de la nouvelle portion de trajectoire déterminée à l'étape 212.

Le procédé de commande peut déterminer une trajectoire complète à l'étape 204 préliminaire de détermination d'au moins une portion de la trajectoire et/ou à chaque étape 212 de détermination d'une nouvelle portion de trajectoire, la trajectoire pouvant ainsi être optimisée pour présenter une durée minimale. Toute trajectoire complète déterminée peut toutefois être modifiée en cours de déroulement du procédé de commande lors d'une nouvelle étape 212 de détermination d'une nouvelle portion de trajectoire.

La détermination d'une nouvelle portion de trajectoire s'effectue grâce aux données disponibles permettant de caractériser l'état en cours, notamment la position courante de la caméra, l'ensemble des tissus reconstruits, l'ensemble des tissus à reconstruire, un modèle 3D prédéfini de la cavité orale, etc.

Si l'unité de commande mettant en oeuvre le procédé 200 de commande utilise un réseau neuronal, le procédé 200 de commande peut comprendre une étape préliminaire 216 d'entraînement du réseau neuronal à partir de données provenant de trajectoires mises en oeuvre par des opérateurs humains expérimentés sur un grand nombre d'arcades dentaires différentes. Le réseau neuronal ainsi entraîné permet la mise en oeuvre de l'étape 212 de détermination de la ou les portions de trajectoires en fonction du statut courant du procédé.

Selon un autre mode de réalisation de l'invention, l'étape 206 de commande est réalisée par l'utilisation de règle heuristiques prédéterminées et ajustées en fonction de l'état en cours.

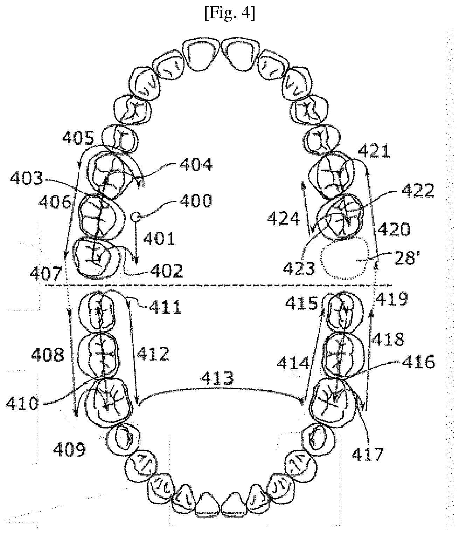

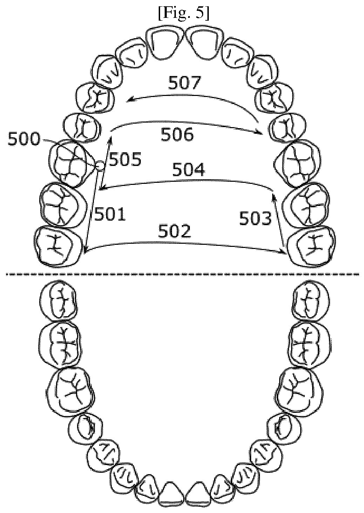

Les figures 4 et 5 représentent schématiquement des exemples de trajectoires pouvant être suivies par la caméra intra-orale installée sur le bras robotisé commandé par l'unité de commande, en suivant le procédé de commande selon l'invention.

La représentation de l'arcade dentaire est analogue à celle représenté dans la figure 1, et les dents sont identifiées par leur référence en fonction de la notation de la Fédération Dentaire Internationale représentée sur cette figure 1 et ne sont pas représentées sur les figures 4 et 5 pour des raisons de clarté.

La figure 4 représente une première trajectoire comprenant plusieurs phases représentant des portions de trajectoires, l'objectif de cette trajectoire étant par exemple la reconstruction des tissus formant l'ensemble des molaires de l'arcade dentaire, d'un patient ou d'un modèle 3D physique. L'ensemble des tissus des molaires forment ainsi l'ensemble des tissus à reconstruire et cet ensemble est défini automatiquement par un dispositif informatique, manuellement par un opérateur humain, ou de manière semi-automatique par un opérateur humain assisté d'un dispositif informatique.

Le bras robotisé ou un opérateur humain mène la caméra vers une position 400 de départ, qui peut être quelconque. Ici, la position de départ est au niveau de la face palatine de la deuxième molaire 17 droite. La capture d'image commence et la caméra suit une première portion 401 de trajectoire au niveau des faces palatines jusqu'à la molaire 18. La caméra suit ensuite une deuxième portion 402 de trajectoire jusqu'à atteindre la face occlusale de la molaire 18, puis une troisième portion 403 de trajectoire pour scanner les faces occlusales des molaires 18, 17, et 16. La face palatine de la molaire 16 ne faisant pas partie des tissus reconstruit mais faisant partie des tissus à reconstruire, une quatrième portion 404 de trajectoire est suivie par la caméra jusqu'à atteindre cette face.

La caméra suit ensuite les trajectoires suivantes :
- une cinquième portion 405 de trajectoire jusqu'à atteindre la face vestibulaire de la molaire 16,
- une sixième portion 406 de trajectoire pour capturer les images des faces vestibulaires des molaires jusqu'à atteindre la molaire 18,
- une septième portion 407 de trajectoire jusqu'à atteindre les dents mandibulaires,
- une huitième portion 408 de trajectoire pour capturer les images des faces vestibulaires des molaires 48, 47 et 46,
- une neuvième portion 409 de trajectoire jusqu'à atteindre la face occlusale de la molaire 46,
- une dixième portion 410 de trajectoire pour capturer les images des faces occlusales des molaires 46, 47 et 48,
- une onzième portion 411 de trajectoire pour atteindre la face linguale de la molaire 48,
- une douzième portion 412 de trajectoire pour capturer les images des faces linguales des molaires 48, 47 et 46,
- une treizième portion 413 de trajectoire jusqu' à atteindre la face linguale de la molaire 36,
- une quatorzième portion 414 de trajectoire pour capturer les images des faces linguales des molaires 36, 37 et 38,
- une quinzième portion 415 de trajectoire jusqu'à atteindre la face occlusale de la molaire 38,
- une seizième portion 416 de trajectoire pour capturer les images des faces occlusales des molaires 38, 37 et 36,
- une dix-septième portion 417 de trajectoire jusqu'à atteindre la face vestibulaire de la molaire 36,
- une dix-huitième portion 418 de trajectoire pour capturer les images des faces vestibulaires des molaires jusqu'à atteindre la molaire 38,
- une dix-neuvième portion 419 de trajectoire jusqu'à atteindre les dents maxillaires.

À l'arrivée au niveau de l'emplacement 28' de la molaire 28, le procédé de commande détecte l'absence de cette molaire. Les tissus de la molaire 28 peuvent donc être supprimés de l'ensemble des tissus à reconstruire et la trajectoire ou la portion de trajectoire suivante peut être recalculée. En particulier, une vingtième portion 420 de trajectoire est suivie pour capturer les images des faces vestibulaires des molaires 27 et 28, une vingt-et-unième portion 421 de trajectoire permet d'atteindre la face occlusale de la molaire 26, une vingt-deuxième portion 422 de trajectoire permet de capturer les images des faces occlusales des molaires 26 et 27. La molaire 28 étant absente, la vingt-troisième portion 423 de trajectoire permet d'atteindre la face palatine de la molaire 27 et la vingt-quatrième portion 424 permet de capturer les images des faces palatines des molaires 27 et 26.

Le procédé atteint ensuite une étape finale dans lequel la caméra intraorale est retirée de la cavité orale.

La figure 5 représente une deuxième trajectoire comprenant plusieurs phases représentant des portions de trajectoires, l'objectif de cette trajectoire étant par exemple la reconstruction du palais d'un patient ou d'un modèle 3D physique.

Le bras robotisé ou un opérateur humain mène la caméra vers une position 500 de départ, qui peut être quelconque. Ici, la position de départ est au niveau de la face palatine de la première molaire 16 droite. La capture d'image commence et la caméra suit une première portion 501 de trajectoire au niveau des faces palatines jusqu'à la molaire 18. La caméra suit ensuite une deuxième portion 502 de trajectoire pour capturer des images du palais jusqu'à atteindre la face palatine de la molaire 28 de sorte à parcourir le palais dans sa largeur. La détection de cette face palatine par segmentation sémantique permet de valider que toute la largeur du palais a été capturée.

La caméra suit ensuite une troisième portion 503 de trajectoire de sorte à se placer au niveau des faces palatines des molaires 26 et 27 et une quatrième portion 504 de trajectoire permet de capturer de nouvelles images du palais jusqu'à atteindre les molaires maxillaires droite 17 et 18. La caméra se déplace ensuite dans selon une cinquième portion 505 de trajectoire de sorte à atteindre la face palatine de la prémolaire 15 et parcourir de nouveau la largeur du palais dans une sixième portion 506 de trajectoire jusqu'à atteindre la face palatine de la prémolaire 24, puis de suivre une septième portion 507 de trajectoire pour terminer la capture du palais au niveau des canines et incisives maxillaires. À chaque parcours de la largeur du palais, la détection des dents permet de s'assurer que la largeur du palais à bien été traversée.

Le procédé atteint ensuite une étape finale dans lequel la caméra intraorale est retirée de la cavité orale.

Cette trajectoire est purement à but illustratif et le nombre de traversées transversales nécessaires à la capture des images de l'ensemble du palais peut être supérieur ou inférieur, par exemple selon la taille du palais ou le champ de vision de la caméra.

De même, d'autres objectifs peuvent être mis en oeuvre, notamment l'ensemble des tissus à reconstruire peut être l'ensemble des tissus de l'arcade dentaire de sorte à réaliser une reconstruction complète de l'arcade dentaire.

## Revendications

1. Procédé de commande d'un bras (102) robotisé comprenant une caméra (104) configurée pour la capture d'images intraorales, le procédé comprenant une étape (204, 212) de détermination d'au moins une portion d'une trajectoire dudit bras robotisé à l'intérieur d'une cavité orale, une étape (206) de commande du déplacement du bras robotisé dans la cavité orale en fonction de ladite portion de trajectoire déterminée, et une étape (208) de capture d'images de la cavité orale par la caméra du bras robotisé,
**caractérisé en ce que** le procédé comprend en outre les étapes suivantes :
- une étape (202) préliminaire de définition d'un ensemble de tissus intra-oraux dont au moins une image doit être captée par la caméra, dit ensemble de tissus à reconstruire,
- une étape (204) préliminaire de détermination d'au moins une portion de la trajectoire du bras robotisé en fonction d'une position de départ de la caméra et de l'ensemble de tissus à reconstruire,
- une étape (210) de segmentation sémantique de chaque pixel de chaque image capturée de sorte à déterminer un ensemble de tissus reconstruits parmi l'ensemble de tissus à reconstruire,
- après chaque ajout d'un tissu dans l'ensemble de tissus reconstruits, une étape (212) de détermination d'une nouvelle portion de trajectoire en fonction de l'ensemble de tissus reconstruits et l'ensemble de tissus à reconstruire.

2. Procédé de commande selon la revendication 1, **caractérisé en ce que** la trajectoire ou la portion de trajectoire est déterminée en fonction d'une trajectoire modèle prédéfinie correspondant à au moins une trajectoire classique utilisée par un opérateur humain.

3. Procédé de commande selon la revendication 1, **caractérisé en ce qu'**il comprend une étape (216) préalable d'entraînement d'un réseau neuronal d'une unité de commande du robot par apprentissage par renforcement, ladite étape préalable d'entraînement prenant comme données d'entrée une pluralité de trajectoires classiques utilisées par un opérateur humain.

4. Procédé de commande selon l'une des revendications 1 à 3, **caractérisé en ce que** la trajectoire ou la portion de trajectoire est déterminée en fonction d'un modèle 3D prédéfini de la cavité orale.

5. Procédé de commande selon l'une des revendications 1 à 4, **caractérisé en ce qu'**au moins une portion de trajectoire comprend un déplacement jusqu'à une détection d'un tissu prédéterminé par la caméra (104) intraorale, ledit tissu prédéterminé étant détecté par segmentation sémantique dans au moins une image de ladite caméra intraorale (104).

6. Procédé de commande selon l'une des revendications 1 à 5, **caractérisé en ce qu'**il comprend une étape de détermination d'une nouvelle trajectoire suite à une détection d'un tissu non prévu par la caméra (104) intraorale, ledit tissu non prévu étant détecté par segmentation sémantique dans au moins une image de ladite caméra intraorale.

7. Procédé de commande selon l'une des revendications 1 à 6, **caractérisé en ce que** l'ensemble de tissus à reconstruire comprend au moins un tissu inclus dans la liste suivante :
- une dent,
- une gencive,
- un palais,
- une prothèse dentaire,
- une préparation dentaire,
- un pilier implantaire.

8. Système d'imagerie comprenant un bras (102) robotisé comprenant une caméra (104) configurée pour la capture d'images intraorales, **caractérisé en ce qu'**il comprend une unité (106) de commande, configurée pour recevoir les images capturées par la caméra (104) intraorale, et commander le bras (102) robotisé selon un procédé (200) de commande selon l'une des revendications 1 à 7.
